# EUROPEAN PATENT SPECIFICATION

(11) **EP 1 289 449 B1**
(45) Date of publication and mention of the grant of the patent: **08.03.2006**
(21) Application number: 01940977.0
(22) Date of filing: 13.06.2001
(51) Int. Cl.: A61F 2/06

(54) **TWO BALLOON CATHETERS FOR STAGED STENT EXPANSION**
ZWEI BALLONKATHETER ZUR STUFENWEISEN EXPANDIERUNG EINES STENTS
DILATATION DE STENT EN DEUX TEMPS AU MOYEN DE DEUX BALLONNETS

(30) Priority: 14.06.2000 US 211642 P
(43) Date of publication of application: 12.03.2003
(73) Proprietor: MEDINOL LTD., Tel Aviv 61581 (IL)
(72) Inventor: RICHTER, Jacob, 47226 Ramat Hasharon (IL)
(74) Representative: Kuhnen & Wacker
(86) International application number: PCT/IL2001/000543
(87) International publication number: WO 2001/095833

(56) References cited:
- EP-A- 0 903 121
- WO-A-00/03662
- WO-A-96/38109
- WO-A-99/12601

## Description

### FIELD OF THE INVENTION

The present invention relates generally to catheter balloons for implanting stents. More particularly, the present invention relates to a catheter balloon which utilizes two balloons coaxially disposed within one another.

### BACKGROUND OF THE INVENTION

It is well known to use a balloon catheter to intraluminally deliver and implant a stent. Typically, to implant a stent with a balloon catheter, the unexpanded stent is disposed around the deflated balloon of a balloon catheter. The balloon is then delivered to the desired implantation site and inflated. The inflation of the balloon expands the stent, implanting it at the desired location.

One shortcoming of conventional balloon catheters is that they may cause the ends of the stent to flare out during implantation. This flaring out is referred to as "dogboning". Dogboning causes at least two undesirable effects. First, dogboning exacerbates any foreshortening of the stent during expansion. Second, dogboning causes the edges of the end of the stent to project in a direction perpendicular to the wall of the vessel in which the stent is being implanted. These projecting edges potentially increase trauma to the wall of the lumen. Documents WO 00 03662, WO 99 12601, WO 96 38109, and EP-A-0903121 describe combinations of a balloon catheter and a stent according to the preamble of claim 1.

### SUMMARY OF THE INVENTION

Embodiments of the present invention include a balloon catheter with two balloons . An inner balloon is shorter than an outer balloon, and also shorter than a stent which is to be implanted. The outer balloon overlays the inner balloon, and is longer that the stent.

To implant the stent, the catheter is delivered to a desired site in a vessel. Pressure is applied to the inner balloon, inflating the balloon and implanting the central portion of the stent. Further increases in pressure rupture the inner balloon. Because the outer balloon overlays the inner balloon, the pressure inflates the outer balloon, expanding the remainder of the stent. The balloons may then be deflated and removed, leaving the implanted stent in the vessel.

### BRIEF DESCRIPTION OF THE DRAWINGS

Fig. 1 shows a cross-sectional view of an embodiment of a catheter balloon assembly constructed according to the principles of the present invention which is in the deflated condition;
Fig. 2 shows a cross-sectional view of the catheter of Fig. 1 after partial inflation; and
Fig. 3 shows a cross-sectional view of the catheter of Fig. 1 after full inflation.

### DETAILED DESCRIPTION OF THE INVENTION

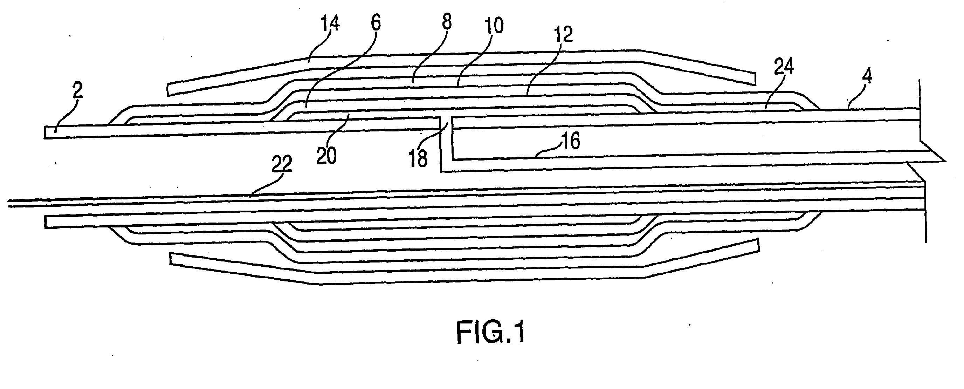

Fig. 1 shows a schematic view of an embodiment of a catheter balloon constructed in accordance with the principles of the present invention. The details of the catheter have not been included here, as they are well known to those skilled in the art. The precise configurations of the catheter shaft, guidewire lumen, and inflation lumen can be chosen as desired. For example, the catheter may be designed as a rapid-exchange system or as an over-the-wire system. The balloon catheter includes a catheter shaft 2. An inner balloon 6 is sealed to the outer surface 4 of the catheter shaft 2. The length of the inner balloon is chosen so that it is less than the length of the stent which it is designed to implant. The inner balloon 6 may be formed of a non-compliant material.

An outer balloon 8 is disposed around the inner balloon 6. The inner surface 10 of the outer balloon is immediately adjacent to the outer surface of the inner balloon. The two surfaces are permitted to move with respect to each other. The outer balloon 8 is sealed to the outer surface 4 of the catheter shaft 2 at the ends of the balloon. The outer balloon 8 may be formed of a non-compliant material. In the illustrated embodiment, the length of the outer balloon is chosen so that it is approximately 4 mm longer than the stent. In this and other embodiments, when the stent is crimped around the deflated balloon, the same amount of balloon may extend past the stent on each side - - i.e. the balloon may, for example, extend past the stent by 2mm on each side.

An inflation lumen 16 is located within the catheter shaft 4. The inflation lumen 16 is in fluid communication with the interior 20 of the inner balloon 6 through an aperture 18 in the catheter shaft 4. A pressurized medium, such as saline, may be introduced into the inflation lumen 16 to inflate the inner balloon. The space between the inner balloon and the outer balloon is not provided with an inflation lumen.

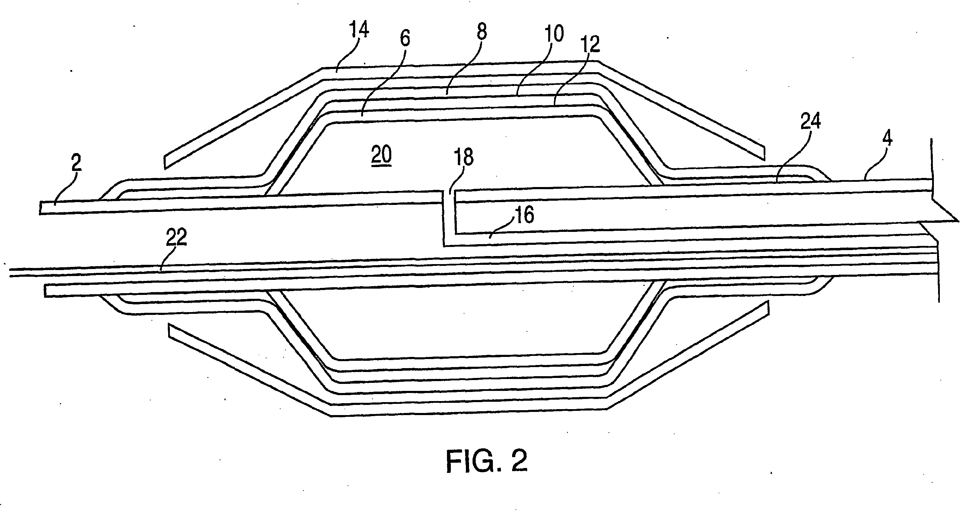

In operation, a guidewire 22 may be routed to the desired inflation location. The balloon catheter, the catheter shaft 4 of which has a guidewire port located adjacent the balloons, with a crimped stent may be then placed over the guidewire 22 and delivered to the desired location. A pressurized medium is introduced into the inflation lumen. The pressurized medium passes into the interior 20 of the inner balloon 6, and begins to inflate the inner balloon 6. The inner balloon 6 applies pressure to both the stent 14 and the outer balloon 8. Typically, at approximately 304 or 406 kPa (3 or 4 atmospheres) (depending on the particular stent design chosen), the stent 14 begins to expand. As shown in Fig. 2, because the inner balloon 6 is shorter than the stent 14, only the middle portion of the stent 14 begins to expand. At, for example, approximately 507 kPa (five atmosphere), the stent 14 is sufficiently expanded so that it is implanted into the vessel wall.

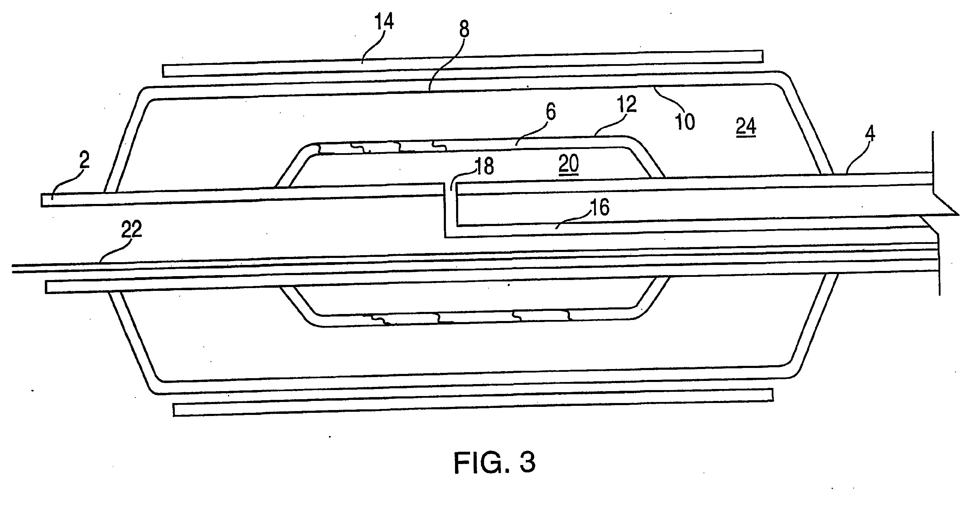

As shown in Fig. 3, upon further application of the pressurized medium to the inflation lumen, the inner balloon 6 ruptures. The burst pressure of the inner balloon may be less than 1010 kPa (10 atmospheres), and in some embodiments be approximatelyl 507 kPa (5 atmosphere), for example. Rupture of the inner balloon 6 allows fluid communication between the inflation lumen 16 and the cavity formed between the outer balloon and the inner balloon.

When further pressure is applied to the inflation lumen, the outer balloon expands the entire length of the stent. The operator may then apply as much pressure as desired, up to the burst pressure of the outer balloon to firmly implant the stent. Typically, the burst pressure of the outer balloon should be greater than that of the inner balloon. Thus, for example, in a particular embodiment the burst pressure of the inner balloon may be selected to be, for example, 507 kPa (5 atmospheres) and the burst pressure of the outer balloon may be selected to be equal to 1010 kPa (10 atmosphere), i.e.,to give an approximately 507kPa (5 atmosphere) difference.

The balloons may then be deflated, and the catheter and guidewire may then by removed.

In the foregoing specification, the invention has been described with reference to specific exemplary embodiments. It will, however, be evident that various modifications and changes may be made thereunto without departing from the scope of the invention as set forth in the appended claims. The specification and drawings are accordingly to be regarded in an illustrative rather than a restrictive sense.

## Claims

1. The combination of a stent and balloon catheter comprising:
a balloon catheter with a first (6) and a second (8) balloon, the second balloon (8) overlaying the first balloon (6), the second balloon(8)having a length which is greater than the length of the first balloon(6); and
an expandable stent (14) mounted over the first and second catheter balloons, **characterised in that** the burst pressure of the first balloon (6) is less than the burst pressure of the second balloon (8).

2. The combination according to claim 1 wherein the burst pressure of the inner balloon (6) is less than 1010kPa (10 atmospheres).

3. The combination according to claim 1 or 2 wherein the burst pressure of the inner balloon (6) is approximately 507kPa (5 atmospheres).

4. The combination according to claim 1, 2 or 3 wherein the burst pressure of the outer balloon (8) is greater than 1010kPa (10 atmospheres).

5. The combination according to any of claims 1-4 wherein the outer balloon (8) is formed of a non-compliant material.

6. The combination according to any of claims 1-5 wherein the inner balloon(6) is formed of a non-compliant material.

7. The combination according to any of claims 1-6 wherein the catheter shaft(2) has a guidewire port located adjacent the balloons.

8. The combination according to any of claims 1-7 wherein the burst pressure of the first balloon (6) is at least 507 kPa (5 atmospheres) less than the burst pressure of the second balloon (8).

9. The combination according to any of claims 1-8 wherein the length of the first balloon (6) is less than the length of the stent(14).

10. The combination according to any of claims 1-9 wherein the length of the second balloon(8)is greater than the length of the stent(14).

## Patentansprüche

1. Kombination eines Stents und eines Ballonkatheters, aufweisend:
einen Ballonkatheter mit einem ersten (6) und einem zweiten (8) Ballon, wobei der zweite Ballon (8) über dem ersten Ballon (6) liegt, wobei der zweite Ballon (8) eine Länge aufweist, welche größer ist als die Länge des ersten Ballons (6); und
einen expandierbaren Stent (14), montiert über dem ersten und zweiten Katheterballon, **dadurch gekennzeichnet, dass**
der Berstdruck des ersten Ballons (6) geringer ist als der Berstdruck des zweiten Ballons (8).

2. Kombination nach Anspruch 1, wobei der Berstdruck des inneren Ballons (6) geringer als 1010 kPa (10 Atmosphären) ist.

3. Kombination nach Anspruch 1 oder 2, wobei der Berstdruck des inneren Ballons (6) ungefähr 507 kPa (5 Atmosphären) ist.

4. Kombination nach Anspruch 1, 2 oder 3, wobei der Berstdruck des äußeren Ballons (8) größer als 1010 kPa (10 Atmosphären) ist.

5. Kombination nach irgendeinem der Ansprüche 1 bis 4, wobei der äußere Ballon (8) aus einem unnachgiebigen Material ausgebildet ist.

6. Kombination nach irgendeinem der Ansprüche 1 bis 5, wobei der innere Ballon (6) aus einem unnachgiebigen Material ausgebildet ist.

7. Kombination nach irgendeinem der Ansprüche 1 bis 6, wobei der Katheterschaft (2) eine Führungsdrahtöffnung aufweist, lokalisiert angrenzend an die Ballone.

8. Kombination nach irgendeinem der Ansprüche 1 bis 7, wobei der Berstdruck des ersten Ballons (6) zumindest 507 kPa (5 Atmosphären) geringer als der Berstdruck des zweiten Ballons (8) ist.

9. Kombination nach irgendeinem der Ansprüche 1 bis 8, wobei die Länge des ersten Ballons (6) geringer ist als die Länge des Stents (14).

10. Kombination nach irgendeinem der Ansprüche 1 bis 9, wobei die Länge des zweiten Ballons (8) größer ist als die Länge des Stents (14).

## Revendications

1. La combinaison d'un stent et d'un cathéter à ballonnet, comprenant :
un cathéter à ballonnet avec un premier (6) et un second (8) ballonnet, le second ballonnet (8) recouvrant le premier ballonnet (6), le second ballonnet (8) ayant une longueur qui est supérieure à la longueur du premier ballonnet (6) ; et
un stent expansible (14) monté sur les premier et second cathéters à ballonnet, **caractérisé en ce que**
la pression d'éclatement du premier ballonnet (6) est inférieure à la pression d'éclatement du second ballonnet (8).

2. La combinaison de la revendication 1, où la pression d'éclatement du ballonnet intérieur (6) est inférieure à 1010 kPa (10 atm).

3. La combinaison de la revendication 1 ou 2, où la pression d'éclatement du ballonnet intérieur (6) d'environ 507 kPa (5 atm).

4. La combinaison de la revendication 1, 2 ou 3, où la pression d'éclatement du ballonnet extérieur (8) est supérieure à 1010 kPa (10 atm).

5. La combinaison de l'une des revendications 1 à 4, où le ballonnet extérieur (8) est formé d'un matériau non compliant.

6. La combinaison de l'une des revendications 1 à 5, où le ballonnet intérieur (6) est formé d'un matériau non compliant.

7. La combinaison de l'une des revendications 1à 6, où la tige du cathéter (2) possède un orifice pour fil de guidage, situé adjacent aux ballons.

8. La combinaison de l'une des revendications 1 à 7, où la pression d'éclatement du premier ballonnet (6) est inférieure d'au moins 507 kPa (5 atm). à la pression d'éclatement du second ballonnet (8).

9. La combinaison de l'une des revendications 1 à 8, où la longueur du premier ballonnet (6) est inférieure à la longueur du stent (14).

10. La combinaison de l'une des revendications 1 à 9, où la longueur du second ballonnet (8) est supérieure à la longueur du stent (14).
